# FASCICULE DE BREVET EUROPEEN

(11) **EP 2 099 565 B1**
(45) Date de publication et mention de la délivrance du brevet: **14.12.2016**
(21) Numéro de dépôt: 07871864.0
(22) Date de dépôt: 14.12.2007
(51) Int. Cl.: B01J 23/85, B01J 23/882, B01J 37/02, C10G 47/12, B01J 31/02, B01J 31/28, B01J 31/34, B01J 37/20, C10G 45/08, C10G 47/16

(54) **CATALYSEUR D'HYDROTRAITEMENT, SON PROCÉDÉ DE PRÉPARATION ET SON UTILISATION**
HYDRIERUNGSKATALYSATOR, VERFAHREN ZU DESSEN HERSTELLUNG UND DESSEN VERWENDUNG
HYDROTREATMENT CATALYST, METHOD FOR ITS PRODUCTION AND USE THEREOF

(30) Priorité: 22.12.2006 FR 0611267
(43) Date de publication de la demande: 16.09.2009
(73) Titulaire: Total Raffinage France, 92400 Courbevoie (FR); IFP Énergies nouvelles, 92852 Rueil-Malmaison Cedex (FR)
(72) Inventeur: BRUN, Claude, 64320 Idron (FR); CHOLLEY, Thierry, 76600 Le Havre (FR); DUPUY, Carole, 1040 Etterbeek (BE); FREMY, Georges, 64390 Sauveterre-de-Béarn (FR); HUMBLOT, Francis, 64300 Lanneplaa (FR)
(74) Mandataire: Largeau, Béatrice
(86) Numéro de dépôt international: PCT/FR2007/002073
(87) Numéro de publication internationale: WO 2008/090282

(56) Documents cités:
- EP-A- 0 506 206
- EP-A1- 0 496 592
- WO-A-2004/067683
- US-A1- 2002 070 147

## Description

La présente invention concerne un catalyseur d'hydrotraitement, son procédé de préparation, son procédé d'activation et l'utilisation de ce catalyseur dans un procédé d'hydrotraitement et/ou d'hydrocraquage d'hydrocarbures, notamment d'hydrocarbures issus de coupes pétrolières de domaine d'ébullition de 40 à 560°C.

Actuellement, la demande en composés hydrocarbonés désulfurés, désazotés et désaromatisés s'accroît et les fabricants développent des catalyseurs de plus en plus efficaces vis-à-vis de la purification des hydrocarbures. Cependant, ces nouveaux catalyseurs sont beaucoup plus coûteux et ne sont accessibles qu'auprès d'un nombre limité de producteurs. En outre, dès leur première régénération, ces catalyseurs présentent une activité souvent bien inférieure à leur activité initiale à l'état neuf dans les mêmes conditions opératoires. Un traitement supplémentaire de réactivation spécifique permet parfois de retrouver une activité proche de l'activité initiale et autorise ainsi la réutilisation du catalyseur pour un nouveau cycle d'hydrodésulfuration profonde et la production de distillats ayant des teneurs en soufre inférieures à 10 ppm. Plusieurs traitements de réactivation sont proposés sur le marché. Ils combinent généralement des étapes de régénération dans des conditions particulières, des traitements chimiques et thermiques, et peuvent être associés à des sulfurations ex situ du catalyseur.

Aujourd'hui, de nombreux catalyseurs « classiques », à base de supports en oxyde(s) réfractaire(s) et contenant une combinaison de métaux des groupes VIB et VIII sont utilisés en raffinerie, à l'état neuf ou régénéré, soit en hydrotraitement, soit en hydrocraquage. S'il s'avère impossible d'augmenter notablement leur activité en désulfuration et/ou en déazotation, ces catalyseurs devront être récupérés, stockés ou détruits, lorsque les spécifications imposées aux carburants deviendront si restrictives qu'il ne sera plus possible de les utiliser. Ce stockage et/ou cette élimination de solides pourraient en outre être soumis à des contraintes environnementales et de sécurité et engendrer des surcoûts importants pour les raffineurs.

La Demanderesse a donc cherché, premièrement, à rendre plus efficaces des catalyseurs connus à base d'oxyde(s) réfractaire(s) et de métaux des groupes VIB et VIII, et de leur conférer des activités en désulfuration et en déazotation au moins équivalentes à celles des meilleurs catalyseurs du marché, et, deuxièmement, à améliorer l'activité de catalyseurs d'hydrotraitement régénérés afin d'augmenter le nombre de cycles de recyclage et de retarder leur mise au rebut et leur destruction.

Les catalyseurs d'hydrotraitement ou d'hydrocraquage contenant des composés métalliques, à l'état d'oxydes ou sous d'autres formes, pour être actifs, doivent nécessairement être sulfurés avant utilisation. Cette sulfuration peut être faite soit in *situ* dans le réacteur d'hydrotraitement de la raffinerie, soit ex *situ.* La sulfuration peut être réalisée au moyen de sulfure d'hydrogène, de mercaptans, de sulfures organiques, de polysulfures et/ou de soufre élémentaire, ces composés étant introduits seuls, en mélange avec un solvant ou en même temps que la charge.

Avant cette étape de sulfuration, certains de ces catalyseurs sont préalablement modifiés par un traitement avec des composés organiques chélatants ou complexants.

La sulfuration et la modification préalable peuvent se faire *in situ,* c'est-à-dire dans le réacteur d'hydrotraitement/hydroconversion, ou bien *ex situ,* c'est-à-dire dans un réacteur dédié. On peut également envisager une modification préalable *ex situ* combinée à une sulfuration *in situ* dans le réacteur d'hydrotraitement/hydroconversion.

La gamme des composés organiques chélatants ou complexants utilisables est assez large. Il est ainsi connu de modifier ces catalyseurs au moyen d'acides de type thioglycolique ou encore de thioalcools, de composés thioacétoniques et de thiodiazoles ou encore de thiocyanates tels que proposés notamment par les demandes suivantes : EP 289211, EP 300629, EP 338788 , EP 357295, EP 456592, EP 478365 et EP 506206. D'autres catalyseurs ont été modifiés par traitement avec des composés organiques alcool-acides (EP 482817), de mono-, di- ou polyalcools éventuellement étherifiés (EP 601722, US 3954673, US 4012340, WO 01/76741), de composés de types urée, polyamines, EDTA, hydrazine et autres composés azotés (EP 181035, EP 335754, EP 1043069, WO 01/76741, US 3954673 et US 4012340). Des catalyseurs modifiés avec des monoesters en C₂₋₁₄ sont décrits dans les demandes de brevet EP 466 568 et EP 1046424.

Le document US 2002/0070147 décrit un procédé de désulfuration utilisant un catalyseur contenant un acide mercapto carboxylique.

Les demandes de brevet WO 2006/077302 et WO 2006/077326 de là demanderesse décrivent l'utilisation de certaines molécules destinées à augmenter l'activité.

Tous ces composés visent à améliorer l'efficacité des catalyseurs en hydrotraitement, plus particulièrement en hydrodésulfuration. Cependant ces modifications ne permettent pas toujours d'accroître suffisamment les performances du catalyseur pour faire face aux spécifications concernant les teneurs en soufre des carburants qui ne cessent de devenir de plus en plus contraignantes pour les raffineurs. Ainsi par exemple, selon les Directives du Parlement Européen et du Conseil Européen, les pays de la Communauté Européenne doivent produire du carburant diesel contenant moins de 10 ppm à l'horizon 2008 - 2011 alors que la norme 2005 est de 50 ppm. De même, pour tous les pays d'Amérique du Nord, la teneur en soufre du diesel doit passer de 500 ppm à 15 ppm à partir de 2006. Certains pays comme l'Allemagne devancent la législation européenne et imposent d'ores et déjà la vente de diesel à moins de 10 ppm. Des contraintes similaires s'appliquent déjà sur l'essence et les autres carburants. Cette évolution des spécifications impose des contraintes sur la production des raffineries, contraintes auxquelles les raffineurs doivent se plier au prix d'investissements souvent importants sur les unités d'hydrotraitement ou d'hydrocraquage et/ou, de façon beaucoup plus économique, grâce à l'amélioration des performances en désulfuration des catalyseurs actuels et la possibilité de les recycler plusieurs fois à leur plus haut niveau de performance.

Dans ce but, la Demanderesse a conçu un nouveau type de catalyseur d'hydrotraitement, à base d'oxydes réfractaires et de métaux des groupes VIB et VIII de la classification périodique des éléments, qui présente, après sulfuration, une activité fortement améliorée en désulfuration ainsi qu'en déazotation.

Un catalyseur d'hydrotraitement, comprenant un support à base d'au moins un oxyde réfractaire, au moins un métal du groupe VIII et au moins un métal du groupe VIB de la Classifcation Périodique des Eléments, est caractérisé en ce qu'il comprend en outre au moins un composé organique comportant au moins deux fonctions thiol séparées par au moins un groupement oxygéné de formule (I) :

HS-CxHyOz-SH (I)

dans laquelle x est un nombre entier de 1 à 20, de préférence de 2 à 9, y est un nombre entier de 2 à 60, de préférence de 4 à 12, et z est un nombre entier de 1 à 10, de préférence de 1 à 6, et dans laquelle au moins un groupement oxygéné est une fonction cétone ou éther, de préférence le (ou les) groupement(s) oxygéné(s) est (sont) une fonction cétone ou éther.

Le support à base d'au moins un oxyde réfractaire est généralement de type alumine, silice ou silice-alumine.

Le métal du groupe VIII de la Classification Périodique des Eléments est de préférence choisi parmi le nickel et le cobalt.

Le métal du groupe VIB de la Classification Périodique des Eléments est de préférence le molybdène.

Le groupement oxygéné peut comporter éventuellement des groupements hydrocarbonés latéraux pouvant contenir ou porter un ou plusieurs hétéroatomes choisis parmi N, S et O.

Dans un mode de réalisation préféré du catalyseur selon l'invention au moins un groupement oxygéné est une fonction éther, de préférence le (ou les) groupement(s) oxygéné(s) est (sont) une fonction éther.

Selon l'invention telle que revendiquée, le composé de formule (I) est choisi parmi le 2,2' (mercapto éthyl) éther et le di mercapto 1,8 dioxa octane (ou DMDO). Le composé encore plus préféré de formule (I) est le di mercapto 1,8 dioxa octane (ou DMDO).

Le 2,2' (mercapto éthyl) éther (numéro CAS 2150-02-9) a pour formule développée :

HS - CH₂ - CH₂ - O - CH₂ - CH₂ - SH.

Le di mercapto 1,8 dioxa octane (numéro CAS 14970-87-7) a pour formule développée :

HS-CH₂-CH₂-O-CH₂-CH₂-O-CH₂-CH₂-SH.

En effet, la Demanderesse a établi que la présence sur les catalyseurs d'un composé organique dé formule (I) augmentait l'efficacité en désulfuration et en déazotation des catalyseurs, après activation par sulfuration dans des conditions connues. En utilisant ces nouveaux catalyseurs pour l'hydrotraitement d'hydrocarbures, on arrive, en comparaison à des catalyseurs équivalents n'ayant subi aucune modification préalable, à abaisser la température de réaction de désulfuration d'environ 5 à 25 °C, pour une teneur résiduelle de soufre donnée et toutes conditions opératoires étant identiques par ailleurs (pression, quantité d'hydrogène et vitesse spatiale horaire (VVH)). Un tel gain d'activité permet d'envisager l'obtention de teneurs résiduelles en soufre nettement inférieures à 50 ppm, voire inférieures à 10 ppm, dans les hydrocarbures traités, en faisant varier les conditions opératoires. Si l'unité est déjà en mesure de produire une coupe pétrolière à basse teneur en soufre, ce gain d'activité permettra de réduire la température du réacteur pour produire la teneur en soufre voulue, et de maintenir ainsi l'unité en service pour une durée supplémentaire pouvant atteindre plusieurs mois.

De préférence, le catalyseur comprend de 0,1 à 10 % en poids d'un métal du groupe VIII, de préférence du nickel et/ou du cobalt, et de 1 à 20% en poids d'un métal du groupe VIB, de préférence du molybdène.

Le catalyseur peut être un catalyseur frais, c'est-à-dire non régénéré, provenant directement d'un fabricant, mais le prétraitement selon l'invention par un ou plusieurs composés de formule (I) est également intéressant lorsqu'il s'agit d'un catalyseur régénéré par un traitement chimique ou thermique approprié, par exemple par calcination.

Il est néanmoins connu que certains catalyseurs frais, du fait d'un mode de préparation spécifique, peuvent être plus ou moins réfractaires aux traitements préconisés. De la même manière, pour les mêmes raisons ou suite à des conditions opératoires particulières subies dans l'unité, les catalyseurs régénérés peuvent s'avérer plus ou moins réfractaires à ces traitements.

Le catalyseur selon l'invention contient de préférence au moins 0,001 mole de composé(s) organique(s) de formule (I), en particulier de 0,001 mole à 10 moles, de préférence de 0,01 à 6 moles et de façon encore plus préférée de 0,1 à 3 moles de composé de formule (I) par mole de métaux des groupes VIB et VIII.

L'invention concerne également un procédé de préparation du catalyseur d'hydrotraitement modifié décrit ci-dessus selon la revendication 5. Ce procédé de modification comprend la mise en contact d'un catalyseur comprenant un support à base d'au moins un oxyde réfractaire, au moins un composé d'un métal du groupe VIII, et au moins un composé d'un métal du groupe VIB, avec au moins un composé organique comportant au moins deux fonctions thiol séparées par au moins un groupement oxygéné de formule (I) :

HS-CxHyOz-SH (I)

dans laquelle le compose organique de formule I est choisi parmi le 2,2'(mercapto éthyl)éther et le dimercapto 1,8 dioxa octane (ou AMDO).

De préférence ce procédé de préparation s'applique au cas où le catalyseur comprenant un support à base d'au moins un oxyde réfractaire, au moins un composé d'un métal du groupe VIII, et au moins un composé d'un métal du groupe VIB est un catalyseur régénéré. Dans ce cas le composé de métal est généralement un oxyde.

Selon un mode de réalisation préféré de l'invention, la mise en contact du catalyseur avec le(s) composé(s) de formule (I) se fait en présence d'au moins un solvant et/ou d'au moins un acide.

Lorsque les composés de formule (I) sont liquides à la température d'imprégnation, la mise en contact avec le catalyseur peut se faire en l'absence de solvant. La mise en contact du catalyseur non modifié avec le composé organique de formule (I) se fait de préférence par mise en contact du catalyseur avec une solution contenant l'agent organique. Le volume de solution peut être inférieur, égal ou supérieur au volume poreux du catalyseur. La méthode utilisant un volume de solution inférieur ou égal au volume poreux du catalyseur est parfois appelée « imprégnation à sec ». Lorsque le volume de solution est supérieur au volume poreux du catalyseur, l'excès de solution sera éliminé après l'adsorption du composé organique de formule (I) sur le catalyseur.

Le(s) composé(s) organique(s) de formule (I) est(sont) au moins partiellement soluble(s) dans le solvant utilisé. Le choix du solvant revêt une importance particulière dans la mise en oeuvre du procédé. La sélection du solvant se base sur différents critères tels que son pouvoir solvant des composés de formule (I), son effet dispersant du composé de formule (I), son effet mouillant de la surface du catalyseur et sa disponibilité sur le marché à des conditions économiquement acceptables.

Parmi les solvants qu'on utilisera avantageusement dans l'invention on peut citer l'eau, les fluides supercritiques tels que le dioxyde de carbone, les solvants aromatiques, aliphatiques, alicycliques, les coupes pétrolières, les solvants mono- et polyhydroxylés tels que l'éthanol, le tertio-butanol, l'éthylèneglycol, le diéthylèneglycol, le triéthylèneglycol, le propylèneglycol, le polyéthylèneglycol(PEG), le glycérol, les esters d'alkyle tel que l'acétate d'éthyle, les cétones tels que l'acétone ou la méthyléthylcétone, la N-métylpyrrolidone, les solvants contenant une fonction amide tels que le diméthylacétamide, les solvants contenant une fonction nitrile tel que l'acétonitrile, les carbonates d'alkyle tels que le carbonate d'éthyle, les éthers comme le tetrahydrofurane, des solvants soufrés comme le diméthylsulfoxide et le sulfolane, les acides comme l'acide acétique, et les solvants halogénés, ou un mélange de plusieurs de ces solvants.

Parmi ces solvants, on préfère en particulier l'eau, le toluène, les xylènes, l'éthylèneglycol, le diéthylèneglycol, le triéthylèneglycol, le glycérol, l'éthanol, le tertiobutanol, le polyéthylène glycol (PEG), le plus souvent de poids moléculaire de 200 jusqu'à 1000, le white-spirit et l'éther de pétrole.

Le ou les composés organiques de formule (I) peuvent éventuellement être introduits en présence d'au moins un acide, généralement choisi parmi les acides carboxyliques, les acides hydrocarboxyliques et les polyacides (tels que les acides formique, acétique, glycolique, lactique, tartrique, maléique, citrique, glycérique, gluconique, methoxy-acétique, ethoxy-acétique, malonique, L(+) ascorbique, salicylique, oxalique, orthophtalique, succinique, glyoxylique...), les acides thiocarboxyliques (tels que les acides thiobenzoique, mercapto acétique, 1 et 2 mercapto-propionique, 2-3 dimercapto succinique, mercapto succinique, thioacétique, thioglycolique, thiodiglycolique, dithiodiglycolique...), les acides aminocarboxyliques (tels que l'acide nitrilotriacétique et l'EDTA (acide tétracétique éthylènediamine diammonium)). Parmi ces acides, les acides particulièrement préférés sont les acides lactique, maléique, tartrique, citrique, succinique, oxalique, thioacétique, thioglycolique, nitriloacétique, et l'EDTA.

Le ou les acides peuvent être introduits avec le ou les composés de formule (I) ou séparément, si, par exemple, le mélange obtenu n'est pas homogène. Dans ce cas, il peut être avantageux d'utiliser un solvant permettant l'introduction simultanée du ou des acides avec les composés de formule (I) le solvant assurant alors la dissolution de tous les composés organiques présents. Enfin, il est également possible lorsque le ou les acides sont introduits séparément (avant ou après, de préférence avant), du composé de formule (I) d'utiliser un solvant différent de celui employé pour introduire le composé de formule (I).

La mise en contact du catalyseur avec le(s) composé(s) de formule (I) se fait généralement en présence d'hydrogène et d'au moins un agent de sulfuration choisi de préférence parmi l'hydrogène sulfuré, le soufre élémentaire, le CS₂, les mercaptans, les sulfures et/ou polysulfures, les coupes hydrocarbonées à point d'ébullition inférieur à 400 °C contenant des composés soufrés, généralement au sein d'un réacteur d'hydrotraitement.

La mise en contact du catalyseur, optionnellement préalablement séché de 10 à 600°C, de préférence de 15 à 300°C, et de manière plus préférentielle de 20 à 180°C avec une solution d'un ou plusieurs composés de formule (I) lors de l'étape d'imprégnation peut durer de 1 minute à 7 jours, de préférence au moins 10 minutes à 8 heures, à une température de 0 à 200°C, de préférence à température ambiante (environ 25°C), à une faible pression de la pression atmosphérique à 5 bars, de préférence à pression atmosphérique. Dans le cas où une solution d'acide(s) est introduite avant la solution de composé(s) organique(s) de formule (I), les conditions opératoires de l'imprégnation avec le ou les acides sont similaires à celles de l'imprégnation avec le ou les composés organiques de formule (I). Dans le cas optionnel où le catalyseur est séché, on peut avantageusement profiter de la chaleur résiduelle du catalyseur pour pratiquer une imprégnation à chaud.

A la fin de l'étape d'imprégnation, le catalyseur peut être soumis, éventuellement après élimination d'un excès de solution d'imprégnation, à une étape de maturation optionnelle à une température de 0 à 100°C, de préférence de la température ambiante à 80°C, à pression atmosphérique ou jusqu'aux pressions généralement mise en oeuvre dans les procédés d'hydrotraitement ou d'hydroconversion, dont la durée peut être comprise de quelques minutes à quelques années, dans le cas où le catalyseur resterait stocké en füts. De préférence, l'étape de maturation peut durer de quelques minutes à 2 jours. Un traitement thermique optionnel ou non, consécutif à l'étape de maturation, peut être effectué à des températures de 50°C à 250°C, de préférence de 60 à 180°C, de quelques minutes à plusieurs jours, de préférence de 30 minutes à 3 heures sous atmosphère inerte ou non, avec ou sans débit gazeux, à pression atmosphérique ou jusqu'aux pressions généralement mise en oeuvre dans les procédés d'hydrotraitement ou d'hydroconversion.

Les étapes optionnelles de maturation et de traitement thermique peuvent être omises lorsque le catalyseur est chargé directement dans l'unité d'hydrotraitement ou d'hydroconversion.

Le catalyseur modifié par imprégnation avec le ou les composés de formule (I) est parfaitement stable à température ambiante et à l'air. La mise en contact du catalyseur avec le(s) composé(s) de formule (I) peut par conséquent se faire ex situ, c'est-à-dire en dehors du réacteur d'hydrotraitement, et il s'agit là d'un mode de réalisation préféré du procédé selon l'invention. La mise en oeuvre ex situ du procédé selon l'invention peut éventuellement être combinée à une sulfuration ex situ du catalyseur modifié selon l'invention. Ceci permet au raffineur d'acheter un produit immédiatement prêt à l'emploi et de réduire le plus possible les pertes de temps au démarrage de l'unité.

Il est également intéressant de réaliser l'étape de mise en contact du catalyseur par le ou les composés de formule (I) ex situ, et de sulfurer le catalyseur modifié in situ dans le réacteur d'hydrotraitement. Dans ce cas, la mise en place du catalyseur dans l'unité de traitement est plus aisée car le catalyseur non sulfuré, contrairement au catalyseur sulfuré, ne risque pas de s'auto-enflammer.

Une autre variante encore consiste à mettre en oeuvre a la fois la modification du catalyseur et la sulfuration in situ.

Il convient de noter que, même lorsque la modification du catalyseur par le ou les composés de formule (I) se fait in situ dans le réacteur d'hydrotraitement, ces deux opérations constituent généralement deux étapes distinctes réalisées l'une après l'autre, la mise en contact avec le composé de formule (I) précédant l'étape de sulfuration. Mais ces deux étapes peuvent aussi être conduites simultanément.

Outre l'imprégnation des composés organiques dans les pores d'un catalyseur frais ou régénéré, il est également possible d'introduire lesdits composés organiques au cours de la fabrication/mise en forme du catalyseur. On peut par exemple incorporer le ou les composés organiques de formule (I) au support avant même le dépôt des composés des métaux des groupes VIB et VIII. Ceci peut être fait par mélange d'un ou de plusieurs composés organiques avec les composants du support avant la mise en forme de celui-ci, ou encore par imprégnation du support mis en forme avec les composés organiques.

Une autre possibilité consiste à introduire les composés organiques et les composés des métaux des groupes VIB et VIII simultanément, soit en les mélangeant aux composants du support avant mise en forme, soit par imprégnation d'un support déjà mis en forme avec à la fois les composés organique et les sels des métaux des groupes VIB et VIII. Les composés des métaux peuvent être des sels des métaux, des oxydes des métaux, ou d'autres formes de composés. L'une ou l'autre des opérations peut être suivie d'un séchage, réalisé dans des conditions telles qu'une partie au moins des composés organiques soit conservé dans le catalyseur.

Il est également possible d'incorporer les composés organiques de formule (I) seulement après de sels des métaux des groupes VIB et VIII. L'une ou l'autre de ces étapes peut éventuellement être suivie d'un séchage et/ou d'une calcination dans des conditions telles qu'une partie au moins des composés soit conservée dans le catalyseur.

Les composés organiques de formule (I) peuvent être introduits dans le catalyseur sous forme liquide et/ou solide particulaire et/ou encore sous forme de solution ou suspension dans un solvant approprié.

L'invention a en outre pour objet un procédé d'activation *in situ* ou *ex situ* d'un catalyseur d'hydrotraitement tel que décrit précédemment. Cette activation se fait par mise en présence du catalyseur et d'hydrogène et/ou d'au moins un agent de sulfuration choisi de préférence parmi l'hydrogène sulfuré, le soufre élémentaire, le CS2, les mercaptans, les sulfures et/ou polysulfures, les coupes hydrocarbonées à point d'ébullition inférieur à 400 °C contenant des composés soufrés. Un agent de sulfuration préféré est le disulfure de diméthyle (DMDS).

L'agent de sulfuration peut être introduit sous forme de gaz ou sous forme diluée dans un solvant, ou comme additif de la charge à hydrotraiter.

L'invention a enfin pour objet l'utilisation du catalyseur ainsi activé *in situ* ou *ex situ,* pour l'hydrotraitement et/ou l'hydrocraquage d'hydrocarbures, en particulier d'hydrocarbures issus de coupes pétrolières de point d'ébullition de 40 à 560°C.

Les exemples donnés dans la suite de la présente description visent à illustrer et non à limiter l'invention.

### Exemple I

Dans le présent exemple, on décrit un mode de préparation d'un catalyseur selon l'invention.

On part d'un catalyseur commercial (catalyseur A), constitué d'une combinaison à 3 % en poids de cobalt et à 10 % en poids de molybdène supporté sur alumine, disponible sur le marché et utilisé communément par les raffineurs dans les unités d'hydrodésulfuration.

La modification de ce catalyseur A par un composé de formule (I) se fait de la manière suivante : 150 g du catalyseur A sont placés dans le ballon d'un évaporateur rotatif mis en rotation à 45 tours par minute. 49,7 g de DiMercaptoDioxaOctane (DMDO) sont injectés en 35 minutes au coeur du catalyseur. Le solide imprégné est laissé pendant 16 heures en rotation lente (20 tours par minutes). On obtient ainsi un catalyseur d'apparence sèche.

Après 20 jours à température ambiante, on charge 100 ml de ce catalyseur dans le réacteur d'une unité pilote de désulfuration de gazole en vue de la réalisation d'un essai d'activité tel que décrit dans l'exemple II ci-après. Après chargement, le catalyseur subit un traitement thermique à 150 °C pendant 16 heures sous un courant d'azote de 20 l/h. Le catalyseur ainsi obtenu est appelé catalyseur B.

### Exemple II

Dans le présent exemple on compare l'activité en hydrodésulfuration et en hydrodéazotation du catalyseur B ayant subi une modification selon l'invention (Exemple I) à celle du catalyseur A n'ayant subi aucun traitement de modification.

Chacun des catalyseurs A (non modifié) et B (modifié selon l'invention) est sulfuré par un gazole additivé à 2% en poids de disulfure de diméthyle (DMDS) selon une procédure recommandée par le fabricant du catalyseur.

Après sulfuration et stabilisation des catalyseurs A et B, on introduit une charge comprenant un mélange de 70 % en poids de gazole et 30 % en poids d'une coupe hydrocarbonée de type LCO (*Light Cycle Oil*) issue du craquage catalytique. Les caractéristiques de cette charge, avant hydrotraitement, sont indiquées dans le Tableau I ci-après.

**TABLEAU I**

| Type de charge | Mélange 30 % LCO-70 % gazole |
|---|---|
| Soufre (ppm) | 9074 |
| Densité à 15 °C (g/ml) | 0,8863 |
| Monoaromatiques (% en poids) | 20,7 |
| Diaromatiques (% en poids) | 21,3 |
| Triaromatiques (% en poids) | 3 ,8 |
| Σ Aromatiques (% en poids) | 45,8 |
| Azote (ppm) | 389 |
| Distillation selon la norme ASTM | |
| D86 (°C) | |
| Point initial | 180 |
| 5% vol | 224 |
| 50% vol | 291 |
| 95 % vol | 360 |
| point final | 361 |

On met en oeuvre la réaction d'hydrotraitement sous une pression de 27.10⁵ Pa (27 bars) avec un rapport hydrogène/hydrocarbures (H₂/HC) de 250 Nl/l à une vitesse spatiale horaire (WH) de 1h⁻¹.

Pour comparer les activités en désulfuration, on ajuste, pour chaque catalyseur, la température réactionnelle à une valeur correspondant à un taux de désulfuration de 99 %. Plus cette température, pour le catalyseur B selon l'invention, est basse en comparaison à la température correspondante pour le catalyseur de référence A, plus ce catalyseur est actif en désulfuration.

Les résultats obtenus sont représentés dans le tableau II sous forme d'écart de température (ΔT) par rapport à la température de référence du catalyseur A (T_{HDS}). Ils correspondent à la température requise pour atteindre un taux de désulfuration de 99 %.

**TABLEAU II**

| Catalyseur | A | B |
|---|---|---|
| HDS | T_{HDS} | T_{HDS} - 4 °C |

On constate que le catalyseur B selon l'invention permet d'atteindre un taux de désulfuration de 99 % à une température inférieure de 4 °C à celle nécessaire pour le catalyseur A. Il a donc une activité en hydrodésulfuration supérieure à celle du catalyseur A

Pour comparer les activités en déazotation, on mesure, pour chaque catalyseur, la teneur résiduelle en azote pour une température réactionnelle T_{HDN} donnée. Plus cette teneur résiduelle en azote est basse, plus le catalyseur est actif en déazotation.

Les résultats obtenus sont représentés dans le tableau III.

**TABLEAU III**

| Catalyseur | A | B |
|---|---|---|
| N résiduel à T_{HDN} (ppm) | 120 | 81 |

On constate que le catalyseur B selon l'invention permet d'abaisser plus fortement la teneur résiduelle en azote de la charge que le catalyseur A ce qui montre qu'il est également plus actif en déazotation.

## Revendications

1. Catalyseur d'hydrotraitement, comprenant un support à base d'au moins un oxyde réfractaire, au moins un métal du groupe VIII et au moins un métal du groupe VIB de la Classification Périodique des Eléments, **caractérisé en ce qu'**il comprend en outre au moins un composé organique choisi parmi le 2,2' (mercapto éthyl) éther et le di mercapto 1,8 dioxa octane (ou DMDO).

2. Catalyseur selon la revendication précédente, caractérisé en ce le composé organique est le di mercapto 1,8 dioxa octane (ou DMDO).

3. Catalyseur selon l'une des revendications précédentes, comprenant de 0,1 à 10 % en poids d'un métal du groupe VIII, de préférence du nickel et/ou du cobalt, et de 1 à 20% en poids d'un métal du groupe VIB, de préférence du molybdène.

4. Catalyseur selon l'une des revendications précédentes, comprenant au moins 0,001 mole de composé(s) organique(s), en particulier de 0,001 mole à 10 moles, de préférence de 0,01 à 6 moles et de façon encore plus préférée de 0,1 à 3 moles de composé(s) organique(s) par mole de métaux des groupes VIB et VIII.

5. Procédé de préparation d'un catalyseur d'hydrotraitement selon l'une des revendications précédentes, comprenant la mise en contact d'un catalyseur comprenant un support à base d'au moins un oxyde réfractaire, au moins un composé d'un métal du groupe VIII, et au moins un composé d'un métal du groupe VIB, avec au moins un composé organique choisi parmi le 2,2' (mercapto éthyl) éther et le di mercapto 1,8 dioxa octane (ou DMDO).

6. Procédé de préparation selon la revendication 5, **caractérisé par le fait que** le catalyseur comprenant un support à base d'au moins un oxyde réfractaire, au moins un composé d'un métal du groupe VIII, et au moins un composé d'un métal du groupe VIB est un catalyseur régénéré.

7. Procédé de préparation selon l'une des revendications 5 à 6, tel que la mise en contact est suivie d'au moins une étape de maturation, à une température généralement de 0 à 100°C, dont la durée est comprise de quelques minutes à quelques années, ladite étape de maturation étant éventuellement suivie d'au moins une étape de traitement thermique, à une température de 50 à 250°C, et de durée de quelques minutes à plusieurs jours.

8. Procédé de préparation selon l'une des revendications 5 à 7, **caractérisé par le fait que** la mise en contact du catalyseur avec ledit/lesdits composé(s) organique(s) se fait en présence d'au moins un solvant et/ou d'au moins un acide.

9. Procédé de préparation selon la revendication précédente, **caractérisé par le fait que** le(s) composé(s) organique(s) est (sont) au moins partiellement soluble(s) dans le solvant utilisé.

10. Procédé de préparation selon la revendication 8 ou 9, **caractérisé par le fait que** le solvant est choisi parmi l'eau, le toluène et les xylènes, l'éthylèneglycol, le diéthylèneglycol, le triéthylèneglycol, le glycérol, l'éthanol, le tertiobutanol, le polyéthylèneglycol (PEG), le plus souvent de poids moléculaire de 118 (le triéthylèneglycol) jusqu'à 1000, le white-spirit et l'éther de pétrole.

11. Procédé de préparation selon l'une des revendications 5 à 10, **caractérisé par le fait que** la mise en contact du catalyseur avec le(s)dit(s) composé(s) organique(s) se fait ex situ, en dehors d'un réacteur d'hydrotraitement.

12. Procédé de préparation selon l'une des revendications 5 à 11, **caractérisé par le fait que** la mise en contact du catalyseur avec le(s)dit(s) composé(s) organique(s) se fait en présence d'hydrogène et d'au moins un agent de sulfuration choisi de préférence parmi l'hydrogène sulfuré, le soufre élémentaire, le CS₂, les mercaptans, les sulfures et/ou polysulfures, les coupes hydrocarbonées à point d'ébullition inférieur à 400 °C contenant des composés soufrés, généralement au sein d'un réacteur d'hydrotraitement.

13. Procédé d'activation *in situ* ou *ex situ* d'un catalyseur d'hydrotraitement selon l'une quelconque des revendications 1 à 4 ou préparé selon l'une des revendications 5 à 12, **caractérisé par le fait que** ledit catalyseur est mis en présence d'hydrogène et d'au moins un agent de sulfuration choisi de préférence parmi l'hydrogène sulfuré, le soufre élémentaire, le CS₂, les mercaptans, les sulfures et/ou polysulfures, et les coupes hydrocarbonées à point d'ébullition inférieur à 400 °C contenant des composés soufrés, l'agent de sulfuration étant de préférence le disulfure de diméthyle.

14. Utilisation du catalyseur d'hydrotraitement activé par un procédé selon la revendication précédente, pour l'hydrotraitement et/ou l'hydrocraquage d'hydrocarbures, en particulier d'hydrocarbures issus de coupes pétrolières de point d'ébullition de 40 à 560°C.

## Patentansprüche

1. Hydrotreatingkatalysator, umfassend einen Träger auf Grundlage mindestens eines feuerfesten Oxids, mindestens eines Metalls der Gruppe VIII und mindestens eines Metalls der Gruppe VIB des Periodensystems der Elemente, **dadurch gekennzeichnet, dass** er weiter mindestens eine organische Verbindung, ausgewählt aus 2,2'-(Mercaptoethyl)ether und Dimercapto-1,8-dioxaoctan (oder DMDO), umfasst.

2. Katalysator nach dem vorhergehenden Anspruch, **dadurch gekennzeichnet, dass** die organische Verbindung Dimercapto-1,8-dioxaoctan (oder DMDO) ist.

3. Katalysator nach einem der vorhergehenden Ansprüche, umfassend von 0,1 bis 10 Gew.-% eines Metalls der Gruppe VIII, vorzugsweise Nickel und/oder Kobalt, und von 1 bis 20 Gew.-% eines Metalls der Gruppe VIB, vorzugsweise Molybdän.

4. Katalysator nach einem der vorhergehenden Ansprüche, umfassend mindestens 0,001 Mol der organischen Verbindung(en), insbesondere von 0,001 Mol bis 10 Mol, vorzugsweise von 0,01 bis 6 Mol und noch mehr bevorzugt von 0,1 bis 3 Mol der organischen Verbindung(en) pro Mol der Metalle der Gruppen VIB und VIII.

5. Verfahren zur Herstellung eines Hydrotreatingkatalysators nach einem der vorhergehenden Ansprüche, umfassend das Inkontaktbringen eines Katalysators, umfassend einen Träger auf Grundlage mindestens eines feuerfesten Oxids, mindestens einer Verbindung eines Metalls der Gruppe VIII und mindestens einer Verbindung eines Metalls der Gruppe VIB, mit mindestens einer organischen Verbindung, ausgewählt aus 2,2'-(Mercaptoethyl)ether und Dimercapto-1,8-dioxaoctan (oder DMDO).

6. Herstellungsverfahren nach Anspruch 5, **gekennzeichnet durch** die Tatsache, dass der Katalysator, umfassend einen Träger auf Grundlage mindestens eines feuerfesten Oxids, mindestens einer Verbindung eines Metalls der Gruppe VIII und mindestens einer Verbindung eines Metalls der Gruppe VIB, ein regenerierter Katalysator ist.

7. Herstellungsverfahren nach einem der Ansprüche 5 bis 6, **dadurch gekennzeichnet, dass** das Inkontaktbringen von mindestens einem Schritt des Alterns bei einer Temperatur im Allgemeinen von 0 bis 100°C, dessen Dauer von einigen Minuten bis einigen Jahren reicht, gefolgt wird, wobei der Schritt des Alterns gegebenenfalls von mindestens einem Schritt des Wärmebehandelns bei einer Temperatur von 50 bis 250°C und mit einer Dauer von einigen Minuten bis mehreren Tagen gefolgt wird.

8. Herstellungsverfahren nach einem der Ansprüche 5 bis 7, **gekennzeichnet durch** die Tatsache, dass das Inkontaktbringen des Katalysators mit der/den organischen Verbindung(en) in Gegenwart mindestens eines Lösemittels und/oder mindestens einer Säure erfolgt.

9. Herstellungsverfahren nach dem vorhergehenden Anspruch, **gekennzeichnet durch** die Tatsache, dass die organische(n) Verbindung(en) zumindest teilweise in dem verwendeten Lösemittel löslich ist (sind).

10. Herstellungsverfahren nach Anspruch 8 oder 9, **gekennzeichnet durch** die Tatsache, dass das Lösemittel aus Wasser, Toluol und Xylolen, Ethylenglycol, Diethylenglycol, Triethylenglycol, Glycerol, Ethanol, tert-Butanol, Polyethylenglycol (PEG), üblicherweise mit einem Molekulargewicht von 118 (Triethylenglycol) bis 1000, White Spirit und Petrolether ausgewählt ist.

11. Herstellungsverfahren nach einem der Ansprüche 5 bis 10, **gekennzeichnet durch** die Tatsache, dass das Inkontaktbringen des Katalysators mit der/den organischen Verbindung(en) ex situ, außerhalb eines Hydrotreatingreaktors, erfolgt.

12. Herstellungsverfahren nach einem der Ansprüche 5 bis 11, **gekennzeichnet durch** die Tatsache, dass das Inkontaktbringen des Katalysators mit der/den organischen Verbindung(en) in Gegenwart von Wasserstoff und mindestens eines Sulfidierungsmittels, vorzugsweise ausgewählt aus Schwefelwasserstoff, elementarem Schwefel, CS₂, Mercaptanen, Sulfiden und/oder Polysulfiden, Kohlenwasserstofffraktionen mit einem Siedepunkt unterhalb von 400°C, enthaltend Schwefelverbindungen, im Allgemeinen im Inneren eines Hydrotreatingreaktors erfolgt.

13. Verfahren zur *in situ-* oder *ex situ*-Aktivierung eines Hydrotreatingkatalysators nach einem der Ansprüche 1 bis 4 oder hergestellt nach einem der Ansprüche 5 bis 12, **gekennzeichnet durch** die Tatsache, dass der Katalysator mit Wasserstoff und mindestens einem Sulfidierungsmittel, vorzugsweise ausgewählt aus Schwefelwasserstoff, elementarem Schwefel, CS₂, Mercaptanen, Sulfiden und/oder Polysulfiden und Kohlenwasserstofffraktionen mit einem Siedepunkt unterhalb von 400°C, enthaltend Schwefelverbindungen, versetzt wird, wobei das Sulfidierungsmittel vorzugsweise Dimethyldisulfid ist.

14. Verwendung eines Hydrotreatingkatalysators, aktiviert durch ein Verfahren nach dem vorhergehenden Anspruch, zum Hydrotreating und/oder Hydrocracking von Kohlenwasserstoffen, insbesondere von Kohlenwasserstoffen aus Erdölfraktionen mit einem Siedepunkt von 40 bis 560°C.

## Claims

1. A hydrotreating catalyst comprising a support based on at least one refractory oxide, at least one metal of Group VIII and at least one metal of Group VIB of the Periodic Table of the Elements, **characterized in that** it further includes at least one organic compound chosen from 2-mercaptoethyl ether and 1,8-dimercaptodioxaoctane (DMDO).

2. The catalyst as claimed in the preceding claim, **characterized in that** the organic compound is 1,8-dimercaptodioxaoctane (DMDO).

3. The catalyst as claimed in one of the preceding claims, comprising 0.1 to 10% by weight of a metal of Group VIII, preferably nickel and/or cobalt, and 1 to 20% by weight of a metal of Group VIB, preferably molybdenum.

4. The catalyst as claimed in one of the preceding claims, comprising at least 0.001 mol of organic compound(s), in particular 0.001 mol to 10 mol, preferably 0.01 to 6 mol and even more preferably 0.1 to 3 mol of organic compound(s) per mole of metals of Groups VIB and VIII.

5. A method of preparing a hydrotreating catalyst as claimed in one of the preceding claims, which comprises bringing a catalyst, comprising a support based on at least one refractory oxide, at least one compound of a metal of Group VIII and at least one compound of a metal of Group VIB, into contact with at least one organic compound chosen from 2-mercaptoethyl ether and 1,8-dimercaptodioxaoctane (DMDO).

6. The method of preparation as claimed in claim 5, **characterized in that** the catalyst comprising a support based on at least one refractory oxide, at least one compound of a metal of Group VIII and at least one compound of a metal of Group VIB is a regenerated catalyst.

7. The method of preparation as claimed in one of claims 5 to 6, such that the contacting step is followed by at least one maturation step at a temperature generally from 0 to 100°C, the duration of which is from a few minutes to a few years, said maturation step being optionally followed by at least one heat treatment step at a temperature of 50 to 250°C and of a duration of a few minutes to a few days.

8. The method of preparation as claimed in one of claims 5 to 7, **characterized in that** the step of bringing the catalyst into contact with said organic compound(s) takes place in the presence of at least one solvent and/or at least one acid.

9. The method of preparation as claimed in the preceding claim, **characterized in that** the organic compound(s) is (are) at least partly soluble in the solvent used.

10. The method of preparation as claimed in either of claims 8 and 9, **characterized in that** the solvent is chosen from water, toluene, xylenes, ethylene glycol, diethylene glycol, triethylene glycol, glycerol, ethanol, *tert*-butanol, polyethylene glycol (PEG), usually with a molecular weight ranging from 118 (triethylene glycol) up to 1000, white spirit and petroleum ether.

11. The method of preparation as claimed in one of claims 5 to 10, **characterized in that** the step of bringing the catalyst into contact with said organic compound(s) takes place *ex situ,* outside a hydrotreatment reactor.

12. The method of preparation as claimed in one of claims 5 to 11, **characterized in that** the step of bringing the catalyst into contact with said organic compound(s) takes place in the presence of hydrogen and at least one sulfurizing agent preferably chosen from hydrogen sulfide, elemental sulfur, CS₂, mercaptans, sulfides and/or polysulfides and hydrocarbon fractions having a boiling point below 400°C and containing sulfur-containing compounds, generally within a hydrotreatment reactor.

13. A method for the *in situ* or *ex situ* activation of a hydrotreating catalyst as claimed in any one of claims 1 to 4 or prepared as claimed in one of claims 5 to 12, **characterized in that** said catalyst is brought into the presence of hydrogen and at least one sulfurizing agent preferably chosen from hydrogen sulfide, elemental sulfur, CS₂, mercaptans, sulfides and/or polysulfides and hydrocarbon fractions having a boiling point below 400°C and containing sulfur-containing compounds, the sulfurizing agent preferably being dimethyl disulfide.

14. The use of the hydrotreating catalyst activated by a method as claimed in the preceding claim, for the hydrotreatment and/or hydrocracking of hydrocarbons, in particular hydrocarbons resulting from petroleum fractions having a boiling point ranging from 40 to 560°C.
